Europäisches Patentamt

European Patent Office

Office européen des brevets

(19)

(11) Veröffentlichungsnummer: **0 154 879 B1**

# (12) EUROPÄISCHE PATENTSCHRIFT

(45) Veröffentlichungstag der Patentschrift: **29.01.92**

(21) Anmeldenummer: **85102059.4**

(22) Anmeldetag: **25.02.85**

(51) Int. Cl.⁵: **A61L 2/10**

(54) **Vorrichtung zum Entkeimen der Innenflächen von Rohrstrecken für Flüssigkeiten oder Gase.**

(30) Priorität: **24.02.84 DE 3406780**

(43) Veröffentlichungstag der Anmeldung:
**18.09.85 Patentblatt 85/38**

(45) Bekanntmachung des Hinweises auf die
Patenterteilung:
**29.01.92 Patentblatt 92/05**

(84) Benannte Vertragsstaaten:
**AT BE CH DE FR GB IT LI LU NL SE**

(56) Entgegenhaltungen:
**EP-A- 0 085 504**
**GB-A- 1 250 829**
**US-A- 2 352 787**

(73) Patentinhaber: **Eisenwerke Fried.Wilh. Düker
GmbH & Co.**

**W-8782 Karlstadt(DE)**

(72) Erfinder: **Dietz, Josef, Dipl.-Ing.**
**Blumenstrasse 21**
**W-8750 Aschaffenburg(DE)**
Erfinder: **Noll, Werner**
**Aschaffenstrasse 52**
**W-8750 Aschaffenburg(DE)**

(74) Vertreter: **Patentanwälte Leinweber & Zimmermann**
**Rosental 7/II Aufg.**
**W-8000 München 2(DE)**

EP 0 154 879 B1

# Beschreibung

Die Erfindung betrifft eine Vorrichtung zum Entkeimen der Innenflächen von Rohrstrecken in Rohrnetzen für Flüssigkeiten oder Gase.

Zum Entkeimen der Innenflächen derartiger Rohrstrecken ist es bekannt, sie mit einer Desinfektionslösung zu füllen. Da eine derartige Desinfektionslösung, z.B. eine Chlorlösung, stark gesundheitsschädlich ist, muß ein Eindringen der Desinfektionslösung in das in Betrieb befindliche angrenzende Rohrnetz unter allen Umständen verhindert werden. Das bedeutet, daß alle Absperrorgane, die den zu desinfizierenden Leitungsabschnitt von den weiter genutzten Versorgungssträngen abtrennen, vorher auf Dichtigkeit geprüft werden müssen. Der Umgang mit derartigen Desinfektionslösungen erfordert daher die Beachtung strenger Sicherheitsvorkehrungen. Ein weiterer Nachteil solcher Desinfektionslösungen, wie z.B. Chlorlösungen, besteht darin, daß sie mitunter zu einer Korrosion der Rohrleitungen und der Armaturen führen.

Die Beseitigung der Desinfektionslösungen nach abgeschlossener Desinfektion hat sich als sehr problematisch und aufwendig erwiesen. Außerdem muß die betreffende Rohrstrecke anschließend solange mit Wasser gespült werden, bis die Desinfektionslösung vollständig ausgespült ist. Trotz verringerter Konzentration der Desinfektionslösung beim Spülvorgang bringt dessen Beseitigung doch einige Probleme mit sich.

Der Erfindung liegt die Aufgabe zugrunde, eine gattungsgemäße Vorrichtung zu schaffen, mit der sich die Innenflächen von Rohrstrecken in Rohrnetzen kostengünstig und schnell entkeimen lassen.

Die erfindungsgemäße Vorrichtung, mit der diese Aufgabe gelöst wird, ist im wesentlichen gekennzeichnet durch einen Grundkörper, der gegebenenfalls selbstfahrend ausgebildet oder über ein an seinem vorderen Ende befestigtes Zugseil an der Rohrinnenfläche entlang beweglich ist und an dem mindestens eine in einer Fassung angeordnete UV-Lampe festgelegt ist. Mit Hilfe dieser Vorrichtung ist es erstmals möglich, nach Verlegung von Rohren und vor deren Inbetriebnahme die Rohrstrecke zu entkeimen, ohne zu diesem Zweck in umständlicher und kostspieliger Weise ein Auffüllen der Rohre mit einer aggressiven Desinfektionslösung und eine nachfolgende Spülung vornehmen zu müssen. Die Vorteile der erfindungsgemäßen Vorrichtung machen sich insbesondere dann bemerkbar, wenn Rohrstrecken mit großem Innendurchmesser in der Größenordnung von 1000 mm zu entkeimen sind.

An dieser Stelle ist zu erwähnen, daß es zum Entkeimen von Flüssigkeiten oder Gasen, beispielsweise Wasser und Luft, bereits lange bekannt ist, eine in einem Rohr stationär gelagerte UV-Lampe zu verwenden, an der die zu entkeimende Flüssigkeit oder das Gas vorbeigeleitet wird. Ferner sind stationäre Einrichtungen bekannt (GB-A-1250829), mit deren Hilfe Behälter bzw. Rohrstükke, d.h. in ihren Abmessungen sehr beschränkte Hohlkörper, mittels einer in sie eingeführten UV-Lampe bestrahlt und auf diese Weise sterilisiert werden können. Auffallend ist die baulich sehr aufwendige Ausgestaltung dieser bekannten Einrichtungen, die erforderlich ist, um allein derart räumlich begrenzte Hohlkörper zu entkeimen.

Ähnlich kompliziert ist eine weiterhin bekannte Einrichtung zum Entkeimen von Gefäßen, beispielsweise Flaschen ausgebildet (US-A-2 352 787).

Anzumerken ist schließlich noch, daß zum Überprüfen des baulichen Zustands von Rohrstrecken Inspektionsfahrzeuge bekannt sind (EP-A-85 504), mit deren Hilfe das Innere einer Pipeline inspiziert werden kann. Die Fortbewegung erfolgt mit Hilfe kleiner Räder, die an federbelasteten Führungsarmen befestigt sind. Auch diese Fahrzeuge vermochten es nicht, dem Fachmann die erfindungsgemäße Entkeimungsvorrichtung nahezulegen.

Als sehr zweckmäßig zum Schutz und zur Abstützung der UV-Lampen im zu entkeimenden Rohr hat es sich bei der Vorrichtung nach der Erfindung erwiesen, wenn sie in weiterer Ausbildung gekennzeichnet ist durch der Abstützung an der Rohrinnenfläche dienende, sich radial vom Körper fort erstreckende, elastisch nachgiebige Führungen. Diese sorgen im ürigen für eine konzentrierte Lage des Grundkörpers bei dessen Bewegung durch das Rohr unter Zugbeanspruchung des Zugseils. Dieses Zugseil ist vorzugsweise mit dem Stromzuführungskabel der UV-Lampe zusammengefaßt. Selbstverständlich kann der Grundkörper mit einer einzigen UV-Lampe bestückt sein. In diesem Fall muß die nachgiebige Führung zumindest z.T. in Ebenen liegen, die mit der Längsmittelebene des Grundkörpers einen spitzen Winkel einschließen. Auf diese Weise ist sichergestellt, daß sämtliche Rohrwandungsbereiche von der UV-Strahlung erfaßt werden.

In weiterer vorteilhafter Ausgestaltung sind die Führungen des Grundkörpers durch mehrere, an seinem vorderen Endbereich angelenkte, federbelastete Führungsarme gebildet. Die Länge der Führungsarme muß dabei selbstverständlich an die Nennweite der zu entkeimenden Rohrleitung angepaßt sein, damit die gespreizten Führungsarme mit ihren hinteren Enden an der Rohrinnenwand federn anliegen. Die erfindungsgemäße Vorrichtung überwindet aufgrund der federnd angeordneten Führungsarme auch geöffnete Schieber, Kugelhähne, Absperrklappen und Rohrkrümer.

Bei einer in konstruktiver Hinsicht besonders einfachen Ausführungsform der Erfindung umfaßt der Grundkörper eine einzige UV-Lampe, die von einem Schutzring-Käfig umgeben ist. Dabei hat es sich als in baulicher Hinsicht sehr zweckmäßig erwiesen, wenn der Schutzring-Käfig mehrere Schutzringe umfaßt, die in sich parallel zueinander und zu einem Halteflansch erstreckende Ebenen liegen und über Bolzen miteinander bzw. mit dem Flansch verbunden sind. Um sicherzustellen, daß alle Bereiche der Innenflächen von Rohrstrecken von UV-Strahlen erfaßt werden, sind die Bolzen vorzugsweise am Ringumfang zueinander versetzt angeordnet.

Ausführungsbeispiele der Erfindung sind in der Zeichnung dargestellt und werden nachfolgend näher erläutert. Es zeigen:

Fig. 1 eine Vorderansicht einer erfindungsgemäßen Vorrichtung zum Entkeimen der Innenflächen von Rohrstrecken,

Fig. 2 eine Seitenansicht der Vorrichtung nach Fig. 1,

Fig. 3 einen Längsschnitt durch eine abgewandelte Vorrichtung, und

Fig. 4 eine Vorderansicht entsprechend dem Pfeil IV in Fig. 3.

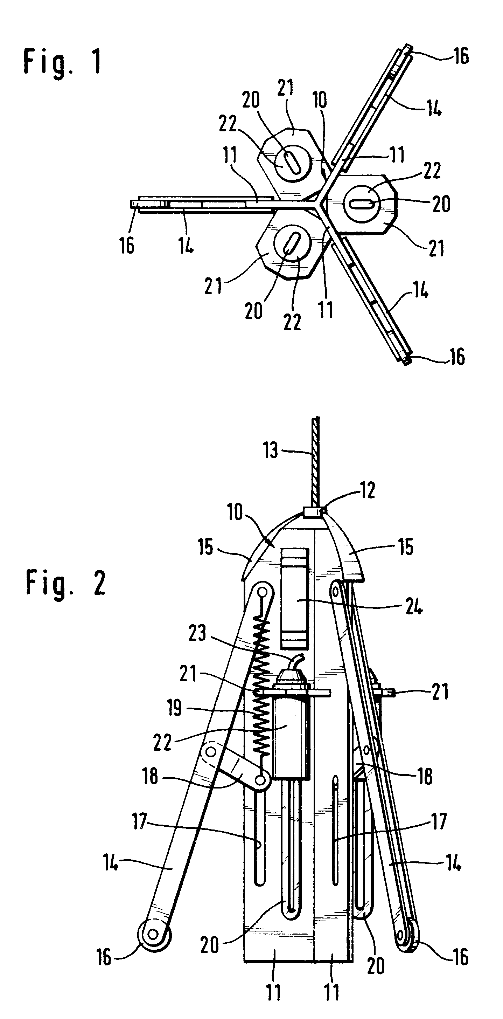

Die in den Fig. 1 und 2 dargestellte erste Ausführungsform der Vorrichtung zum Entkeimen der Innenflächen von Rohrstrecken besteht aus einem Grundkörper 10 mit Y-förmigem Querschnitt, der drei Längsrippen 11 aufweist, die unter einem Winkelabstand von 120° symmetrisch zur Längsachse des Grundkörpers 10 angeordnet sind. Am vorderen Ende des Grundkörpers 10 ist eine Einrichtung 12 zur Befestigung eines Zugseiles 13 angeordnet. Jeder Längsrippe 11 ist ein Führungsarm 14 zugeordnet, der am vorderen Ende angelenkt ist. Damit sich das Gelenk des Führungsarms nicht an der Innenwand der Rohrstrecke verfangen kann, sind am vorderen Ende des Grundkörpers 10 schräge Abweisrampen 15 angeordnet, welche die Längsrippen 11 radial überragen. Am freien hinteren Ende eines jeden Führungsarms 14, das näherungsweise mit dem hinteren Ende des Grundkörpers 10 fluchtet, ist eine Laufrolle 16 drehbar gelagert. In jeder Längsrippe 11 ist ein Längsschlitz 17 vorgesehen, in dem das innere Ende eines Spreizarmes 18 verschiebbar geführt ist, dessen äußeres Ende an dem gegenüberliegenden Führungsarm 14 näherungsweise mittig angelenkt ist. An dem in dem Längsschlitz 17 geführten hinteren Ende eines jeden Spreizarms 18 greift eine Zugfeder 19 an, deren anderes Ende am vorderen Ende der betreffenden Längsrippe 11 befestigt ist. Diese Zugfeder 19 ist daher bestrebt, das innere Ende des Spreizarms 18 nach vorn zu ziehen, um dadurch den zugehörigen Führungsarm 14 vom Grundkörper 10 abzuspreizen. Am Grundkörper 10 können Anschläge vorgesehen sein, um den Spreizwinkel der Führungsarme 14 auf ein geeignetes Maß zu beschränken.

Zwischen benachbarten Längsrippen 11 des Grundkörpers 10 ist jeweils eine UV-Lampe 20 befestigt, und zwar in einer an einem Flansch 21 sitzenden Fassung 22. Die drei UV-Lampen 20 sind dabei zur Längsachse des Grundkörpers 10 symmetrisch angeordnet. Die Stromversorgung erfolgt über Zuleitungen 23 und ein Vorschaltgerät 24 von einem nicht dargestellten Stromzuführungskabel aus, das vorzugsweise mit dem an der Befestigungseinrichtung 12 angreifenden Zugseil 13 zusammengafaßt ist.

Beim Gebrauch wird die Vorrichtung mit dem Zugseil durch die zu entkeimende Rohrleitung gezogen, wobei die Laufrollen 16 der Führungsarme 14 von den Zugfedern 19 an der Innenwand der Rohrleitung federnd in Anlage gehalten werden. Dadurch ist gewährleistet, daß die Längsachse des Grundkörpers 10 zur Rohrmittelachse zentriert ist und alle UV-Lampen von der Innenwand der Rohrleitung den gleichen Abstand aufweisen. Die Innenfläche des Rohrs wird daher von den UV-Lampen 20 gleichmäßig bestrahlt, wodurch bei entsprechender Vorschubgeschwindigkeit eine vollständige Abtötung aller Keime an der Rohrinnenfläche erzielt wird. Aufgrund der federnden Anordnung der Führungsarme 13 kann die Vorrichtung auch geöffnete Schieber, Absperrklappen und Rohrkrümer überwinden.

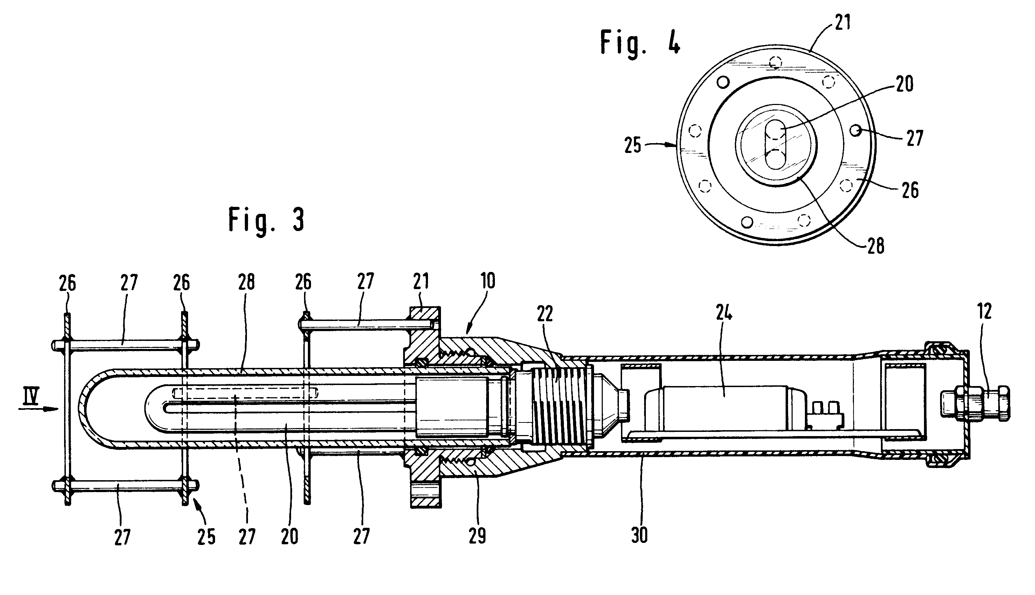

Bei der in den Fig. 3 und 4 veranschaulichten abgewandelten Ausführungsform einfacherer Bauweise ist auf eine Zentrierung der UV-Lampe 20 verzichtet. Anstelle von Spreizarmen, die u.a. sicherstellen, daß keine Lampenberührung mit der Rohrinnenwand eintritt, ist ein Schutzring-Käfig 25 mit mehreren Schutzringen 26 vorgesehen, die in sich parallel zum Flansch 21 erstreckenden Ebenen liegen. Diese Schutzringe 26 sind über am Ringumfang zueinander versetzt angeordnete Bolzen 27 miteinander bzw. mit dem Flansch 21 verbunden. Bei dieser Ausführung ist nur eine UV-Lampe 20 vorgesehen, die sich innerhalb des Schutzring-Käfigs 25 erstreckt, und zwar innerhalb eines sie umgebenden, UV-Strahlen durchlassenden Schutzrohres 28. Außerhalb der Fassung 22 für die UV-Lampe 20 erstreckt sich, ausgehend von einer die Fassung umschließenden und mit dem Flansch 21 verschraubten Buchse 29 ein Rohr 30 aus Kunststoff, das endseitig mit der Einrichtung 12 zur Zugseilbefestigung und zur Durchführung des nicht veranschaulichten Stromzuführungskabels ins Rohrinnere vorgesehen ist. Unmittelbar vor der Fassung 22 ist im Rohr 30 das Vorschaltgerät 24 festgelegt.

Bei dieser einfacheren Ausführung mit nur einer UV-Lampe sorgen die versetzten Bolzen 27 für eine ausreichende UV-Bestrahlung, ohne daß gewisse Bereiche der Rohrinnenwand im Bolzenschatten, d.h. von den Strahlen unerreicht bleiben würden.

Bezugszeichenaufstellung:

| 10 | Grundkörper |
| 11 | Längsrippen |
| 12 | Einrichtung zur Zugseilbefestigung |
| 13 | Zugseil |
| 14 | Führungsarme |
| 15 | Abweisrampen |
| 16 | Laufrollen |
| 17 | Längsschlitz |
| 18 | Spreizarm |
| 19 | Zugfeder |
| 20 | UV-Lampe |
| 21 | Flansch |
| 22 | Fassung |
| 23 | Zuleitung |
| 24 | Vorschaltgerät |
| 25 | Schutzring-Käfig |
| 26 | Schutzring |
| 27 | Bolzen |
| 28 | Schutzrohr |
| 29 | Buchse |
| 30 | Rohr |

**Patentansprüche**

1. Vorrichtung zum Entkeimen der Innenfläche von Rohrstrecken in Rohrnetzen für Flüssigkeiten oder Gase, **gekennzeichnet** durch einen Grundkörper (10), der ggf. selbstfahrend ausgebildet oder über ein an seinem vorderen Ende befestigtes Zugseil (13) an der Rohrinnenfläche entlang beweglich ist und an dem mindestens eine in einer Fassung (22) angeordnete UV-Lampe (20) festgelegt ist.

2. Vorrichtung nach Anspruch 1, gekennzeichnet durch der Abstützung an der Rohrinnenfläche dienende, sich radial vom Körper (10) fort erstreckende, elastisch nachgiebige Führungen (14).

3. Vorrichtung nach Anspruch 2, dadurch gekennzeichnet, daß die Führungen des Grundkörpers (10) durch mehrere, an seinem vorderen Endbereich angelenkte, federbelastete Führungsarme (14) gebildet sind.

4. Vorrichtung nach einem der Ansprüche 1 bis 3, dadurch gekennzeichnet, daß sich die Führungsarme (14) in Längsmittelebenen des Grundkörpers (10) erstrecken und daß die UV-Lampen (20) jeweils zwischen benachbarten Führungsarmen angeordnet sind.

5. Vorrichtung nach einem der Ansprüche 1 bis 4, dadurch gekennzeichnet, daß Federn (19) vorgesehen sind, die jeweils mit dem Grundkörper (10) und einem der Führungsarme (14) gekuppelt sind.

6. Vorrichtung nach Anspruch 1, dadurch gekennzeichnet, daß der Grundkörper (10) eine einzige UV-Lampe (20) umfaßt, die von einem Schutzring-Käfig (25) umgeben ist.

7. Vorrichtung nach Anspruch 6, dadurch gekennzeichnet, daß der Schutzring-Käfig (25) mehrere Schutzringe (26) umfaßt, die in sich parallel zueinander und zu einem Halteflansch (21) erstreckenden Ebenen liegen und über Bolzen (27) miteinander bzw. mit dem Flansch (21) verbunden sind.

8. Vorrichtung nach Anspruch 7, dadurch gekennzeichnet, daß die Bolzen (27) am Ringumfang zueinander versetzt angeordnet sind.

9. Vorrichtung nach Anspruch 8, dadurch gekennzeichnet, daß der Flansch eine zentrische Bohrung aufweist, durch die sich die UV-Lampe (20) hindurch zu der Fassung (22) erstreckt.

10. Vorrichtung nach Anspruch 9 , dadurch gekennzeichnet, daß die UV-Lampe (20) von einem UV-Strahlen durchlassenden Schutzrohr (28) umgeben ist, das in der zentrischen Bohrung des Flansches (21) gelagert ist.

**Claims**

1. Device for disinfecting the internal surface of lengths of piping in pipe systems for liquids or gases, characterised by a main body (10) which is possibly arranged to be self-propelled or is movable along the internal surface of the pipe by means of a draw rope (13) secured to its front end, and on which there is secured at least one ultra-violet lamp (20) arranged in a holder (22).

2. Device according to claim 1, characterised by elastically yieldable guides (14) which are used for bearing against the internal surface of the pipe and which extend radially from the body (10).

3. Device according to claim 2, characterised in that the guides of the main body (10) are constituted by a plurality of spring-loaded guide arms (14) pivotally attached to its front end region.

**4.** Device according to one of claims 1 to 3, characterised in that the guide arms (14) are disposed in longitudinal central planes of the main body (10), and that the ultra-violet lamps (20) are arranged each between adjacently situated guide arms.

**5.** Device according to one of claims 1 to 4, characterised in that springs (19) are provided which are each coupled to the main body (10) and to one of the guide arms (14).

**6.** Device according to claim 1, characterised in that the main body (10) has a single ultra-violet lamp (20), which is surrounded by a protective ring cage (25).

**7.** Device according to claim 6, characterised in that the protective ring cage (25) comprises a plurality of protective rings (26) which are situated in planes parallel to one another and to a holding flange (21), and which are connected to one another and to the flange (21) respectively by means of pins (27).

**8.** Device according to claim 7, characterised in that the pins (27) are arranged offset with respect to one another at the ring periphery.

**9.** Device according to claim 8, characterised in that the flange has a central bore through which the ultra-violet lamp (20) extends to the holder (22).

**10.** Device according to claim 9, characterised in that the ultra-violet lamp (20) is surrounded by a protective tube (28) which allows the passage of ultra-violet radiation and which is mounted in the central bore of the flange (21).

**Revendications**

**1.** Dispositif de désinfection des surfaces intérieures de conduites de réseaux de tuyauteries pour des liquides ou des gaz, caractérisé par un corps de base (10), qui est mobile le long de la surface intérieure de la conduite, qu'il soit autopropulseur éventuellement ou tiré par un câble (13) fixé à son extrémité avant et auquel, au minimum, est fixée une lampe UV (20) placée dans une douille (22).

**2.** Dispositif selon la revendication 1, caractérisé par des guides (14) élastiquement déformables, s'éloignant radialement du corps (10), servant à l'appui contre la surface intérieure de la conduite.

**3.** Dispositif selon la revendication 2, caractérisé en ce que les guides du corps de base (10) sont constitués par plusieurs bras de guidage (14) articulés à sa zone d'extrémité avant et commandés par ressort.

**4.** Dispositif selon l'une des revendications 1 à 3, caractérisé en ce que les bras de guidage (14) s'étendent dans des plans médians longitudinaux du corps de base (10) et en ce que les lampes UV (20) sont respectivement placées entre deux bras de guidage voisins.

**5.** Dispositif selon l'une des revendications 1 à 4, caractérisé en ce que des ressorts (19) sont prévus et respectivement couplés avec le corps de base (10) et l'un des bras de guidage (14).

**6.** Dispositif selon la revendication 1, caractérisé en ce que le corps de base (10) comporte une lampe UV (20), qui est entourée par une cage à anneaux de garde (25).

**7.** Dispositif selon la revendication 6, caractérisé en ce que la cage à anneaux de garde (25) comporte plusieurs anneaux de garde (26), qui se situent dans des plans parallèles entre eux et parallèles à un flasque de maintien (21) et qui sont reliés entre eux et au flasque de maintien (21) par l'intermédiaire de tiges (27).

**8.** Dispositif selon la revendication 7, caractérisé en ce que les tiges (27) sont décalées entre elles sur le périmètre des anneaux.

**9.** Dispositif selon la revendication 8, caractérisé en ce que le flasque présente un alésage central, à travers lequel la lampe UV (20) s'étend jusqu'à la douille (22).

**10.** Dispositif selon la revendication 9, caractérisé en ce que la lampe UV (20) est entourée d'un tube protecteur(28) logé dans l'alésage central du flasque (21) et laissant passer un rayonnement UV.

EP 0 154 879 B1

Fig. 1

Fig. 2

Fig. 4

Fig. 3

EP 0 154 879 B1